Europäisches Patentamt

European Patent Office

Office européen des brevets

(11) Veröffentlichungsnummer: **0 551 860 A1**

# EUROPÄISCHE PATENTANMELDUNG

(21) Anmeldenummer: **93100317.2**

(22) Anmeldetag: **12.01.93**

(51) Int. Cl.$^5$: **A61K  31/55**

(30) Priorität: **13.01.92 DE 4200619**

(43) Veröffentlichungstag der Anmeldung:
**21.07.93 Patentblatt  93/29**

(84) Benannte Vertragsstaaten:
**AT BE CH DE DK ES FR GB GR IE IT LI LU NL PT SE**

(71) Anmelder: **BOEHRINGER INGELHEIM KG**

**W-6507 Ingelheim am Rhein(DE)**

(84) **BE CH DE DK ES FR GR IE IT LI LU NL PT SE AT**

(71) Anmelder: **BOEHRINGER INGELHEIM INTERNATIONAL G.M.B.H.**

**W-6507 Ingelheim am Rhein(DE)**

(84) **GB**

(72) Erfinder: **Kutter, Eberhard, Dr.**
**Am Heerberg 37**
**W-6531 Weiler(DE)**

(54) **PAF-Antagosten als Artzneimitteln zur Behandlung der Dysmenorrhea.**

(57)  Die Erfindung betrifft die Verwendung von 4-(2-Chlorphenyl)-9-methyl-2- [3(4-morpholinyl) -3-propanon-1-yl]-6H-thieno-[3,2-f][1,2,4]triazolo[4,3-a] [1,4]diazepin und 6-(2-Chlorphenyl)-8,9-dihydro-1-methyl-8-[(4-morpholinyl)-carbonyl]-4H,7H-cyclopenta-[4,5]thieno[3,2-f] [1,2,4]triazolo[4,3-a][1,4]diazepin zur Behandlung der Dysmenorrhea, insbesondere der primären Dysmenorrhea.

EP 0 551 860 A1

Die Erfindung betrifft die Verwendung von 4-(2-Chlorphenyl)-9-methyl-2-[3(4-morpholinyl) -3-propanon-1-yl]-6H-thieno-[3,2-f][1,2,4]triazolo[4,3-a] [1,4]diazepin und 6-(2-Chlorphenyl)-8,9-dihydro-1-methyl-8-[(4-morpholinyl)carbonyl]-4H,7H-cyclopenta-[4,5]thieno[3,2-f][1,2,4]triazolo[4,3-a][1,4]diazepin zur Behandlung der Dysmenorrhea, insbesondere der primären Dysmenorrhea.

Aus dem Stand der Technik sind verschiedene Gruppen von Arzneimitteln zur Behandlung der Dysmenorrhea bekannt (Rote Liste 1991). Zum einen werden nichthormonelle Antidysmenorrhoika therapeutisch angewendet, so z.B. Analgetika und Vitamin $B_6$ Komplexe, zum anderen sind hormonelle Arzneimittel, wie z.B. Ethinylestradiol, zur Behandlung von Menstruationsstörungen im Handel. Die Einnahme von Analgetika weist die bekannten Nebenwirkungen auf, wie z.B. die Beeinflussung des Reaktionsvermögens. Die Einnahme von Vitamin $B_6$-Komplexen erweist sich nur dann als sinnvoll, wenn tatsächlich ein Mangel an Vitamin $B_6$ vorliegt. Die Einnahme von Sexualhormonen kann ebenfalls mit einer Reihe von Nebenwirkungen einhergehen, wie z.B. Brustsekretion - und Vergrößerung, Hautausschlag, Eryhtema nodosum, Amenorrhoe sowie einer Verstärkung von Endometriose. Andererseits wurden zur Behandlung von Menstruationsstörungen auch bereits Prostaglandinsynthesehemmer oder Cyclooxygenasehemmer vorgeschlagen; die Nachteile solcher Wirkstoffe - insbesondere das Auftragen von Magen- und Darmulcera sind hinreichend bekannt.

Bei allen diesen Präparaten ist die Wirkung, vor allem bei schweren Formen der Dysmenorrhea, häufig nicht voll befriedigend.

Es war die Aufgabe der vorliegenden Erfindung ein Arzneimittel zur Behandlung der Dysmenorrhea, insbesondere der primären Dysmenorrhea zur Verfügung zu stellen. Es war eine weitere Aufgabe ein Arzneimittel zur Behandlung mit weniger Nebenwirkungen zur Verfügung zu stellen.

Diese Aufgabe wird durch die Verwendung von 4-(2-Chlorphenyl)-9-methyl-2-[3(4-morpholinyl) -3-propanon-1-yl]-6H-thieno-[3,2-f][1,2,4]triazolo[4,3-a] [1,4]diazepin bzw. von 6-(2-Chlorphenyl)-8,9-dihydro-1-methyl-8-[(4-morpholinyl)carbonyl]-4H,7H-cyclopenta[4,5]thieno[3,2-f][1,2,4]triazolo[4,3-a][1,4]diazepin und dessen Enantiomere, insbesondere dem S(-)-Enantiomer gelöst.

Die genannten Verbindungen weisen die für Analgetica bekannten Nebenwirkungen nicht auf. Obwohl die genannten Verbindungen zur chemischen Klasse der Thieno-1,4-diazepine gehören, wurde nachgewiesen, daß sie keine sedierenden Eigenschaften aufweisen. Nebenwirkungen, wie sie bei Sexualhormonen oder Cyclooxygenasehemmern auftreten können, sind bislang nicht bekannt und aufgrund der unterschiedlichen chemischen Strukturen auch nicht zu erwarten.

Eine wirksame orale Einzeldosis beträgt zwischen 40 und 200 mg, bevorzugt 80 - 150 mg, besonders bevorzugt 90 - 120 mg. Die Einnahme des Arzneimittels erfolgt zweckmäßigeweise beim Einsetzen der Menstruationsschmerzen, als wirkungsvoll hat es sich auch erwiesen, wenn mit der Einnahme kurz vor Einsetzen der Menstruation begonnen wird. Im Bedarfsfall kann die Einnahme aufgrund der guten Verträglichkeit der Substanzen wiederholt werden.

Überraschenderweise hat sich gezeigt, daß die erfindungsgemäß vorgeschlagenen Verbindungen auch in besonders schweren Fällen von primärer Dysmenorrhae erfolgreich angewendet werden können.

Am Beispiel des 4-(2-Chlorphenyl)-9-methyl-2-[3(4-morpholinyl)-3-propanon-1-yl]-6H-thieno-[3,2-f]-[1,2,4]triazolo[4,3-a][1,4]diazepins wurde in einer mehrmonatigen Anwendung mit einer Applikation von ca. 80 mg Substanz der Nachweis erbracht, daß die Einnahme der Verbindung rasch zum vollständigen Verschwinden der Menstruationsschmerzen führte.

Die Verbindungen können oral oder parenteral als Zäpfchen verabreicht werden. Die Verbindungen liegen hierbei als aktive Bestandteile in üblichen Darreichungsformen vor, z.B. in Zusammensetzungen, die im wesentlichen aus einem inerten pharmazeutischen Träger und einer effektiven Dosis des Wirkstoffes bestehen, wie z.B. Tabletten, Dragees, Kapseln, Oblaten, Pulver, Lösungen, Suspensionen, Emulsionen, Sirupe, Suppositorien usw. Eine wirksame Dosis der erfindungsgemäßen Verbindungen liegt bei oraler Anwendung zwischen 40 und 200 mg/Dosis. In Notfällen wäre denkbar, die Verbindungen auch i.V. zu applizieren, wobei die Dosierung dann entsprechend geringer als bei oraler Applikation erfolgen muß.

Die genannten Verbindungen sind gut verträglich und praktisch untoxisch sind, so weist beispielsweise die Substanz 4-(2-Chlorphenyl)-9-methyl-2-[3(4-morpholinyl)-3-propanon-1-yl]-6H-thieno-[3,2-f][1,2,4]triazolo-[4,3-a][1,4]diazepin eine $LD_{50}$ von 1960 mg/kg p.o., die Substanz 6-(2-Chlorphenyl)-8,9-dihydro-1-methyl-8-[(4-morpholinyl)carbonyl]-4H,7H-cyclopenta[4,5]thieno-[3,2-f][1,2,4]triazolo[4,3-a][1,4]diazepin eine $LD_{50}$ von 4600 mg/kg p.o. auf.

Die folgenden Beispiele dienen zur näheren Erläuterung der Erfindung.

Herstellung der Wirkstoffe:

2-[4-(2-Chlorphenyl)-9-methyl-6H-thieno[3,2-f][1,2,4]triazolo[4,3-a][1,4]diazepin-2-yl]-ethan-1-carbonsäuremorpholid

5,3 g (0.014 Mol) 2-[4-(2-Chlorphenyl)-9-methyl-6H-thieno-[3,2-f][1,4]diazepin-2-yl]-ethan-1-carbonsäure, 1,8 g N-Hydroxybenztriazol (HOBT) und 60 ml absolutes Dimethylformamid werden unter Rühren bei Raumtemperatur mit 1,2 g (0,014 Mol) Morpholin versetzt, wobei eine klare Lösung erfolgt. Bei 0 - 5°C fügt man anschließend während 5 bis 10 Stunden 3,5 g Dicyclohexylcarbodiimid in fester Form hinzu und hält die Temperatur weitere 6 - 8 Stunden bei 0 - 10°C. Der ausgefallene Dicyclohexylharnstoff wird abgesaugt, mit wenig kaltem Dimethylformamid nachgewaschen und das Filtrat im Vakuum eingedampft. Man löst den Rückstand in Methylenchlorid, wäscht mit 5 %iger Sodalösung und Eiswwasser, dampft die organische Phase ein und bringt den Rückstand im Essigester zur Kristallisation. Ausbeute: 5,2 g (83,2 % d. Th.) farblose Kristalle, Fp. 189 - 190°C.

$^1$H-NMR (CDCl$_3$), $\delta$ = 2,64 (2t, -CH$_2$-CO-), 2,71 (3s, CH$_3$), 3,17 (2t, CH$_2$), 3,33-3,81 (8m, Morpholin), 4,96 (2s, CH$_2$), 6,48 (1s, Thiophen), 7,28-7,60 (4m, Aryl)

B)

3-(Morpholin-4-yl-carbonyl)-5-(2-chlorphenyl)-10-methyl-3,4-dihydro-2H,7H-cyclopenta[4,5]thieno[3,2-f]-[1,2,4]triazolo[4,3-a][1,4]diazepin

15 g (0.038 Mol) 3-Carboxy-5-(2-chlorphenyl)-10-methyl-3,4-dihydro-2H,7H-cyclopenta[4,5]thieno[3,2-f]-[1,2,4]triazolo[4,3-a][1,4]diazepin wurden mit 3,5 g Morpholin mittels Dicyclohexylcarbodiimid in das Amid überführt. Man erhält 15-16 g eines Pulvers vom Fp. 150°C. Aus Ethanol umkristallisiert schmilzt die Verbindung bei 167-168°C. Die Verbindung enthält 1-2% Kristallalkohol.

$^1$H-NMR (CDCl$_3$): $\delta$ 7.22 - 7.59 (m, 4H, Aryl-H); 4.94 (s, breit, 2H, CH$_2$-7-Ring); 2.06 - 4.00 (m, 13H, Cyclopentenyl-H, Morpholin-H); 2.67 (s, 3H, CH$_3$-Triazol-Ring).

Die eingesetzte Säure wird wie folgt hergestellt:

a) 41,6 g (0,27 Mol) Cyclopentanon-3-carbonsäureethylester (H.Stetzer und H. Kuhlmann, Liebigs Ann.Chem. 1979, 944; Kp. 70 - 74°C), 47.8 g o-Chlorcyanoacetophenon und 9 g Schwefel werden in 120 ml Dimethylformamid suspendiert bzw. gelöst und mit 26.5 ml Triethylamin in 120 ml Ethanol versetzt , man erhitzt 30 - 45 Min. auf 30 - 60°C und arbeitet analog Beispiel 1b auf. Nach dem Chromatographieren erhält man 29-30 g 2-Amino-3-(2-chlorbenzoyl)-5-ethoxycarbonyl-5,6-dihydro-4H-cyclopenta[b]-thiophen in Form gelber Kristalle vom Fp.: 121 - 122°C.

b) Bromacetylierung, Umsetzung mit Ammoniak und Cyclisierung in Toluol in Gegenwart von SiO$_2$ ergeben das 7-Carbethoxy-5-(2-chlorphenyl)-7,8-dihydro-3H,6H-cyclopenta[4,5]thieno[2,3-e]-diazepin-2-on als farblose Kristalle vom Fp. 158-160°C.

Das Diazepinon wird weiter umgesetzt. Man erhält den Ester der Triazoloverbindung vom Fp.: 175-176°C und hieraus die entsprechende Carbonsäure durch Verseifung.

Beispiel 1

Tabletten, enthaltend 80 mg Substanz

| Zusammensetzung: | |
| --- | --- |
| Wirkstoff | 80,0 mg |
| Maisstärke | 57,0 mg |
| Milchzucker | 48,0 mg |
| Polyvinylpyrrolidon | 4,0 mg |
| Magnesiumstearat | 1.0 mg |
| | 190,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca.

45°C getrocknet. Das trockne Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.
Tablettengewicht: 190 mg

Beispiel 2

Dragées, enthaltend 50 mg Substanz

| Zusammensetzung: | |
|---|---|
| Wirkstoff | 50,0 mg |
| Maisstärke | 41,5 mg |
| Milchzucker | 30,0 mg |
| Polyvinylpyrrolidon | 3,0 mg |
| Magnesiumstearat | 0,5 mg |
| | 125,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichten aus Zucker und Talkum besteht. Die fertigen Dragées werden mit Wachs poliert.
Dragéegewicht: 175 mg

Beispiel 3

Tabletten, enthaltend 50 mg Substanz

| Zusammensetzung: | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1.5 mg |
| | 200,0 mg |

Herstellung:

Die Substanz, Calciumphosphat, Milchzucker und Maisstärke werden mit einer wässerigen Polyvinylpyrrolidonlösung gleichmäßig befeuchtet. Die Masse wird durch ein Sieb mit 2 mm Maschenweite gegeben, im Umlufttrockenschrank bei 50°C getrocknet und erneut gesiebt. Nach Zumischen des Schmiermittels wird das Granulat auf einer Tablettiermaschine gepreßt.

Beispiel 4

Kapseln, enthaltend 50 mg Substanz

| Zusammensetzung: | |
|---|---|
| Wirkstoff | 50,0 mg |
| Maisstärke | 268,5 mg |
| Magnesiumstearat | 1.5 mg |
| | 320,0 mg |

Herstellung:

Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

Beispiel 5

Suppositorien, enthaltend 50 mg Substanz

| Zusammensetzung: | |
|---|---|
| Wirkstoff | 50 mg |
| Adeps solidus | 1.650 mg |
| | 1.700 mg |

Herstellung:

Das Hartfett wird geschmolzen. Bei 40°C wird die gemahlene Wirksubstanz homogen dispergiert. Es wird auf 38°C abgekühlt und in schwach vorgekühlte Suppositorienformen ausgegossen.

Beispiel 6

Orale Suspension, enthaltend 50 mg Substanz pro 5 ml

| Zusammensetzung: | |
|---|---|
| Wirkstoff | 50 mg |
| Hydroxyethylcellulose | 50 mg |
| Sorbinsäue | 5 mg |
| Sorbit 70%ig | 600 mg |
| Glycerin | 200 mg |
| Aroma | 15 mg |
| Wasser ad | 5 ml |

Herstellung:

Destilliertes Wasser wird auf 70°C erhitzt. Hierin wird unter Rühren Hydroxyethylcellulose gelöst. Nach Zugabe von Sorbitlösung und Glycerin wird auf Raumtemperatur abgekühlt. Bei Raumtemperatur werden Sorbinsäure, Aroma und Substanz zugegeben. Zur Entlüftung der Suspension wird unter Rühren evakuiert.

**Patentansprüche**

1. Verwendung von 4-(2-Chlorphenyl)-9-methyl-2-[3(4-morpholinyl)-3-propanon-1-yl]-6H-thieno-[3,2-f] [1,2,4]triazolo[4,3-a][1,4]diazepin zur therapeutischen Behandlung der Dysmenorrhea, insbesondere der primären Dysmenorrhea.

2. Verwendung von 6-(2-Chlorphenyl)-8,9-dihydro-1-methyl-8-[(4-morpholinyl)carbonyl]-4H,7H-cyclopenta [4,5]thieno-[3,2-f][1,2,4]triazolo[4,3-a][1,4] diazepin zur therapeutischen Behandlung der Dysmenorrhea, insbesondere der primären Dysmenorrhea.

Europäisches Patentamt

**EUROPÄISCHER TEILRECHERCHENBERICHT** Nummer der Anmeldung

der nach Regel 45 des Europäischen Patentübereinkommens für das weitere Verfahren als
europäischer Recherchenbericht gilt

EP    93 10 0317
Seite 1

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| X | EICOSANOIDS<br>Bd. 4, Nr. 3, Oktober 1991,<br>Seiten 137 - 141<br>S.NIGAM ET AL. 'Increased concentrations of eicosanoids and platelet-activating factor in menstrual blood from women with primary dysmenorrhea'<br>* Zusammenfassung *<br>--- | 1-2 | A61K31/55 |
| A | R.BERKOW ET AL., ED. 'the merck manual of diagnosis and therapy, 15. Ausgabe'<br>1987 , MSD RESEARCH LABORATORIES , RAHWAY, N.J., U.S.A.<br>* Seite 1712 - Seite 1713 *<br>---<br><br>-/-- | 1-2 | |

**RECHERCHIERTE SACHGEBIETE (Int. Cl. 5)**

A61K

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| DEN HAAG | 02 APRIL 1993 | THEUNS H.G. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
········································
& : Mitglied der gleichen Patentfamilie, übereinstimmendes Dokument

EPO FORM 1503 03.82 (P04E09)

| | EINSCHLÄGIGE DOKUMENTE | | KLASSIFIKATION DER ANMELDUNG (Int. Cl. 5) |
|---|---|---|---|
| Kategorie | Kennzeichnung des Dokuments mit Angabe, soweit erforderlich der maßgeblichen Teile | Betrifft Anspruch | |
| Y | PROG. CLIN. BIOL. RES. Bd. 308, 1989, Seiten 455 - 462 J.A.BAUER ET AL. 'MODULATION OF RESYNTHESIS OF 1-ALKYL-2-ARACHIDONYL-GLYCERO-3-PHOSPHOCHOLINE AND PHOSPHATIDYLINOSITOLS FOR INTERCEPTION IN VIVO OF FREE ARACHIDONIC ACID, LYSO-PAF, DIACYL-GLYCEROLS, AND PHOSPHOINOSITIDES.' * Zusammenfassung * --- | 1-2 | |
| Y | PROSTAGLANDINS Bd. 32, Nr. 4, Oktober 1986, Seiten 539 - 554 G. MONTRUCCHIO ET AL. 'IN VITRO CONTRACTILE EFFECT OF PLATELET-ACTIVATING FACTOR ON GUINEA-PIG MYOMETRIUM' * Zusammenfassung * --- | 1-2 | |
| Y | PROC. SOC. EXP. BIOL. MED. Bd. 183, Nr. 3, Dezember 1986, Seiten 376 - 381 C. TETTA ET AL. 'PLATELET-ACTIVATING FACTOR CONTRACTS HUMAN MYOMETRIUM IN VITRO' * Zusammenfassung * --- | 1-2 | **RECHERCHIERTE SACHGEBIETE (Int. Cl. 5)** |
| A | CLIN.THER. Bd. 13, Nr. 1, 1991, Seiten 100 - 117 I.HUYBRECHTS 'THE PHARMACOLOGY OF ALPRAZOLAM: A REVIEW' * Seite 106 - Seite 107 * --- | 1-2 | |
| A | DE-A-3 502 392 (BOEHRINGER INGELHEIM KG) * Seite 12 - Seite 13 * & -A- --- | 1-2 | |
| A | DE-A-3 724 031 (BOEHRINGER INGELHEIM KG) * Seite 26 * ----- | 1-2 | |

EPO FORM 1503 03.82 (P04E12)

EP 93 10 0317     -C-

Bemerkung : Obwohl die Ansprüche sich auf ein Verfahren
            im Sinne von Artikel 52(4) EPÜ beziehen,
            wurde die Recherche gegründet auf die ange-
            führten Wirkungen der Verbindungen.